# EUROPEAN PATENT APPLICATION

(11) **EP 4 026 892 A1**
(43) Date of publication of application: **13.07.2022**
(21) Application number: 21151152.2
(22) Date of filing: 12.01.2021
(51) Int. Cl.: C12M 1/36, G05B 11/01

(54) **DEVICE ASSEMBLY AND METHOD FOR CONTROLLING AN INTEGRATED CONTINUOUS PHARMACEUTICAL OR BIOPHARMACEUTICAL MANUFACTURING PROCESS**

(71) Applicant: Sartorius Stedim Biotech GmbH, 37079 Göttingen (DE)
(72) Inventor: Lemke, Johannes, 37079 Göttingen (DE); Matuszczyk, Jens-Christoph, 37079 Göttingen (DE); Höhse, Marek, 37079 Göttingen (DE); Söldner, Robert, 37079 Göttingen (DE); Graf, Alexander, 89077 Ulm (DE)
(74) Representative: Prinz & Partner mbB

(57) **Abstract**

A device assembly for controlling an integrated continuous pharmaceutical or biopharmaceutical manufacturing process comprises: a first process equipment (10) adapted for performing a first process step; a second process equipment (20) adapted for performing a second process step subsequent to the first process step; a single measuring unit (24) adapted for measurement of at least one set of signals of a liquid process medium at a single location, the set of measured signals depending on at least a first parameter and a different second parameter; and an evaluation and control unit (28) adapted for evaluating the set of measured signals to determine a value of the first parameter and a value of the second parameter. The evaluation and control unit (28) is further adapted for determining a first corrective feedback based on the value of the first parameter and a different second corrective feedback based on the value of the second parameter. The evaluation and control unit (28) is further adapted for controlling the first process step by providing the first corrective feedback to the first process equipment (10) and for controlling the subsequent second process step by providing the second corrective feedback to the second process equipment (20).

## Description

The invention relates to a device assembly for controlling an integrated continuous pharmaceutical or biopharmaceutical manufacturing process. The invention also relates to a method for controlling an integrated continuous pharmaceutical or biopharmaceutical manufacturing process.

Continuous manufacturing is becoming more important in the pharmaceutical and especially in the biopharmaceutical industry. This processing strategy is favorable, as it is more efficient, flexible, and has the potential to produce better and more consistent product quality. To develop its full potential, the integration of multiple process steps is necessary, thus eliminating the need for intermediate holding tanks. Thereby facility footprint and processing time are further reduced.

While continuous processing has many benefits, it is also more complex than conventional batch-processing and comes with the increased risk of process deviations, as everything is happening faster, leaving less time to detect, investigate and react to any acute discrepancies or inconsistencies. Traditional off-line analytics (manual sampling followed by sample preparation, measurement and evaluation) are labor-intensive and too slow to enable sufficient control of continuous processes. Therefore, more advanced on-line and/or in-line analytics (automated sampling in a bypass and/or directly in the process line, analyzer close to the process equipment, evaluation and automated feedback control of the process based on the analysis data) is one key to successful continuous manufacturing.

Further, it has to be contemplated that integrated biomanufacturing is just starting to gain traction, with no optimized work flows established yet. Commonly, the measurement and control of two analytes, which is one of the key objects of the present invention, is realized by using multiple sensors in both integrated processes, e.g. one probe in the bioreactor for upstream control and another probe in the chromatography system for downstream control. For example, an on-line glucose sensor, e.g. BioPAT^{®} Trace, could be used to measure and control the glucose concentration in the bioreactor. In the subsequent downstream process for product capture, an in-line UV sensor could be used to monitor the product titer of the perfusion harvest. However, utilization of two online sensors increases costs and setup complexity, and the probability of failure is generally increased if two sensors are used.

With respect to continuous manufacturing, US 9 657 056 B2 describes an integrated and continuous production process for therapeutic protein drug substances. In a production bioreactor in perfusion mode product is secreted into the culture medium and continuously removed from the bioreactor (perfusion harvest). The perfusion harvest is directly fed to a first continuous chromatography system where the product is captured. The eluate from the first chromatography system containing the product is fed into a second chromatography system for further purification.

With respect to sensors and controlling a biopharmaceutical manufacturing process, DE 10 2014 106 916 A1 discloses a device for automated determining of at least two different process parameters from a process fluid. The device includes two different measuring cells, each measuring one process parameter. The device also includes a control and evaluation unit to monitor and/or control the process.

EP 3 243 073 B1, which relates to multi-column chromatography, discloses a method and a setup for controlling at least one parameter of a continuous multi-column chromatography process by using at least one multivariate signal.

It is an object of the invention to simplify the setup and improve efficiency of a continuous pharmaceutical or biopharmaceutical manufacturing process and to improve controlling of such a process.

The above problem is solved by a device assembly according to claim 1 and by a device assembly according to claim 2. The problem is also solved by a method according to claim 10 and by a method according to claim 11. Advantageous and expedient embodiments of the invention are apparent from the respective dependent claims.

According to a first approach, the invention provides a device assembly for controlling an integrated continuous pharmaceutical or biopharmaceutical manufacturing process. The device assembly comprises: a first process equipment adapted for performing a first process step; a second process equipment adapted for performing a second process step subsequent to the first process step; a single measuring unit adapted for measurement of at least one set of signals of a liquid process medium at a single location, the set of measured signals depending on at least a first parameter and a different second parameter; and an evaluation and control unit adapted for evaluating the set of measured signals to determine a value of the first parameter and a value of the second parameter. The evaluation and control unit is further adapted for determining a first corrective feedback based on the value of the first parameter and a different second corrective feedback based on the value of the second parameter. The evaluation and control unit is further adapted for controlling the first process step by providing the first corrective feedback to the first process equipment and for controlling the subsequent second process step by providing the second corrective feedback to the second process equipment.

According to an alternative second approach, device assembly comprises: a first process equipment adapted for performing a first process step; a second process equipment adapted for performing a second process step subsequent to the first process step; a single measuring unit adapted for measurement of at least two different sets of signals of a liquid process medium at a single location, a first set of measured signals depending on at least a first parameter and a different second set of measured signals depending on at least a different second parameter; and an evaluation and control unit adapted for evaluating the first set of measured signals to determine a value of the first parameter and for evaluating the second set of measured signals to determine a value of the second parameter. The evaluation and control unit is further adapted for determining a first corrective feedback based on the value of the first parameter and a different second corrective feedback based on the value of the second parameter. The evaluation and control unit is further adapted for controlling the first process step by providing the first corrective feedback to the first process equipment and for controlling the subsequent second process step by providing the second corrective feedback to the second process equipment.

Before the common basic concept of the two approaches of the invention recited above will be explained, the meanings of some of the terms used herein are clarified below:
An "integrated continuous pharmaceutical or biopharmaceutical manufacturing process" includes several (at least two) process steps which are performed in sequence without interruption. This includes cases where the second process step logically follows the first process step, but in fact both process steps are - at least temporarily - performed concurrently. The term "subsequent" is to be understood accordingly in a logical sense.

A "parameter" is a quantity which changes characteristics of the pharmaceutical or biopharmaceutical manufacturing process. In particular, a parameter can be (i) a process parameter, i.e. a general condition which can be directly set or adjusted, like the temperature of a process medium, or (ii) a process performance indicator or key performance indicator, like viable cell density, which describes the quality of the running process step, or (iii) a critical quality attribute which relates to the quality of the final product, or (iv) an analyte, especially a quantity or a concentration of an analyte in a process medium.

A "single measuring unit" is one functional measuring unit including one measuring cell or probe at one defined location. The single measurement unit performs one measurement of at least one set of signals at a time. Preferably, the single measuring unit records a multivariate signal like a spectrum, but it is also possible that only a signal at or around a single wavelength is measured, for example. Of course, further measuring units can be used in the process device assembly for other purposes.

According to the invention, the measured signals depend on different first and second parameters. This means that the two parameters differ in their impact on the first and second set of signals. In other words: when the value of the first parameter changes, then the measured signals change in accordance with the first parameter's change, and when the value of the second parameter changes, then the measured signals change in accordance with the second parameter's change.

Providing a "corrective feedback" to the first and/or second process equipment does not necessarily mean that a correction is actually necessary. The corrective feedback can also be an information indicating that, currently, no correction is necessary, or, as long as no correction is necessary, no corrective feedback is provided at all. However, in case it is determined that a correction is necessary or expedient, a corresponding information is provided to the concerned process equipment, preferably in real-time.

The invention is based on the finding that in a continuous pharmaceutical or biopharmaceutical manufacturing process a single measuring unit which measures signals at a single location can be utilized for controlling two subsequent process steps. According to the first approach of the invention, it is sufficient to measure at least one set of signals at a time because the set of measured signals depends both on the first parameter which is relevant for controlling the first process step, and on the second parameter which is relevant for controlling the second process step. According to the alternative second approach of the invention, the single measuring unit measures at least two different sets of signals, the first set of measured signals depending on the first parameter and the different second set of measured signals depending on the second parameter.

The concept of the invention is scalable, i.e. it can be expanded to three or more integrated process steps, if circumstances allow.

The general idea behind the invention will be explained by way of a general example and in a comparative manner with reference to previous practice. In a continuous pharmaceutical or biopharmaceutical manufacturing process varying performance in the first process step necessitates a large "safety factor" for the second process step. Since both process steps are integrated into the continuous process, the output of the first process step is at the same time the input of the second process step, without the possibility of analyzing the intermediate product off-line. Due to hardly avoidable biological or process-related fluctuations in the first process step, the second process step must be designed accordingly "generously" in order to absolutely avoid a loss of product. With the device assembly and the method according to the invention it is however possible to control the first process step in order to minimize the fluctuations so that the second process step can be carried out more efficiently. On the other hand, even if the fluctuations of the first process step are not minimized, they can still be detected and considered for controlling the second process step.

Alternative solutions would be to use conventional off-line measurements or to utilize a first on-line sensor specific for a first parameter in the first process step and a second on-line sensor specific for a second parameter in the second process step. While off-line analysis is suitable to monitor robust processes, the time required to retrieve a sample and obtain the results is far too long to allow for adequate control in a continuous process. Intermediate holding tanks would be required to collect the output from the first process step. Only after the results from the respective offline analytics are obtained the process can be continued with the second process step in a batch-wise procedure. This is in contradiction to the desired goal of continuous manufacturing and thus not a practical alternative.

With the device assembly and the method according to the invention, the parameters can be measured on-line or even in-line (automated sampling in the process line) and evaluated in real-time. Accordingly, holding tanks and the associated work effort become obsolete, thus further reducing the footprint and setup complexity. Moreover, the increased risk of contamination in off-line analysis can be avoided. The other alternative, utilization of two different sensors in two process steps, comes with increased setup complexity: footprint is increased due to sensors and additional hardware required; connectivity of each sensor/device to the process control system has to be ensured to enable real-time data monitoring and control; and probability of failure is increased as each sensor/unit comes with individual risks. All this leads to increased costs, with respect to both operational expenditure (opex) and capital expenditure (capex).

The big benefit of continuous manufacturing processes is that the downstream process (i.e. purification of the product of interest) can be started while the upstream process is still running. This has a positive impact especially on labile products. Controlling of the downstream process can be made dependent on the transfer of product from the upstream process to the downstream process, e.g. volumes and corresponding concentrations. This information helps to optimize the utilization of downstream processing capacity. Commonly, chromatography columns are only loaded up to approximately 80 - 90 % to ensure that no product is lost by breakthrough caused by column overload. With the help of an online titer signal in combination with the (known or controlled or calculated) flow rate, for example, the loading of columns can be monitored throughout the running process, thus allowing for more efficient use of column capacity. Of course, this concept can also be applied to other adsorber types or separation techniques (filtration etc.).

As already mentioned, in case the first approach of the invention is used, measuring one set of signals is sufficient to control both process steps. This is possible because the necessary information for determining the corrective feedbacks for both process steps can be derived from the same set of signals which depends on both the first parameter and the second parameter.

In case the second approach of the invention is used, the single measuring unit is preferably adapted for simultaneous measurement of the first and second sets of signals. A close timely correlation of the measurements is advantageous when the second parameter is influenced by the first parameter.

In order to constantly monitor the first and second parameters the single measuring unit is preferably adapted for repeated measurement of the set(s) of signals.

The measuring unit can be arranged in a connecting line (main process line) between the first process equipment and the second process equipment, allowing for in-line analysis. As an alternative, the measuring unit can be arranged in a bypass line (bypassing the main process line) between the first process equipment and the second process equipment for on-line analysis.

In a typical manufacturing process including an upstream process step (cultivation in a bioreactor) and a downstream process step (purification of the product), the measuring unit is preferably arranged in a harvest line downstream of the bioreactor. Usually, at the outlet of the bioreactor a filter or other retaining means are provided to remove unwanted clusters and/or air from the liquid process medium so that the measuring conditions in the harvest line are improved.

However, the measuring unit can also be arranged directly in the bioreactor of the first process equipment. As the perfusion harvest is constantly removed from the bioreactor, the parameters in the harvest are similar to the ones measured in the bioreactor, at least with respect to parameters which are not significantly affected by the filter or retaining means.

According to a preferred embodiment of the invention, the single measurement unit is a (flow-through) measuring cell, preferably including a flow-through measurement chamber, which is part of a spectrometer, preferably based on at least one of the following spectroscopic techniques: scattering, Raman, SERS, MIR, NIR, UV/Vis, fluorescence. Common sensors are often not suitable to quantify two different analytes simultaneously, at least none that are suitable to control two subsequent process steps. In contrast thereto, spectroscopy allows for multiparameter analytics, i.e. simultaneous detection of a plurality of parameters. Moreover, spectroscopy can be implemented in a non-invasive manner, thus further reducing the risk of contamination.

The evaluation and control unit is preferably adapted for applying a first chemometric model and a different second chemometric model to the set(s) of measured signals. Chemometric models are effective software tools for evaluation of the measured signals, especially complex spectral data, with respect to a parameter of interest. Here, a first chemometric model specific for the first parameter and a different second chemometric model specific for the second parameter are used in the evaluation and control unit.

The invention also provides a method for controlling an integrated continuous pharmaceutical or biopharmaceutical manufacturing process, the process comprising at least a first process step performed in or by a first process equipment and a subsequent second process step performed in or by a second process equipment

According to the first approach of the invention, the method comprises the following steps: measuring at least one set of signals of a liquid process medium at a single location, the set of measured signals depending on at least a first parameter and a different second parameter; evaluating the set of measured signals to determine a value of the first parameter and a value of the second parameter; determining a first corrective feedback based on the value of the first parameter and a different second corrective feedback based on the value of the second parameter; controlling the first process step by providing the first corrective feedback to the first process equipment; and controlling the subsequent second process step by providing the second corrective feedback to the second process equipment.

According to the second approach of the invention, the method comprises the following steps: measuring at least two different sets of signals of a liquid process medium at a single location, a first set of measured signals depending on at least a first parameter and a different second set of measured signals depending on at least a different second parameter; evaluating the first set of measured signals to determine a value of the first parameter and for evaluating the second set of measured signals to determine a value of the second parameter; determining a first corrective feedback based on the value of the first parameter and a different second corrective feedback based on the value of the second parameter; controlling the first process step by providing the first corrective feedback to the first process equipment; and controlling the subsequent second process step by providing the second corrective feedback to the second process equipment.

Regarding the advantages and benefits of the method according to the invention, reference is made to the corresponding above statements with respect to the device assembly according to the invention. This also applies to the following aspects of the invention.

In case the first approach of the invention is used, the first and second sets of signals are preferably measured simultaneously during the running process.

The set(s) of signals are preferably measured repeatedly during the running process.

The set(s) of signals are preferably measured in a connecting line, or in a bypass line, between the first process step and the second process step.

According to a preferred embodiment of the invention, the step of measuring set(s) of signals includes measuring a spectrum of the liquid process medium, preferably at least one of the following spectra: scattering, Raman, SERS, MIR, NIR, UV/Vis, fluorescence.

The step of evaluating the set(s) of signals includes applying a first chemometric model and a different second chemometric model to the set(s) of measured signals.

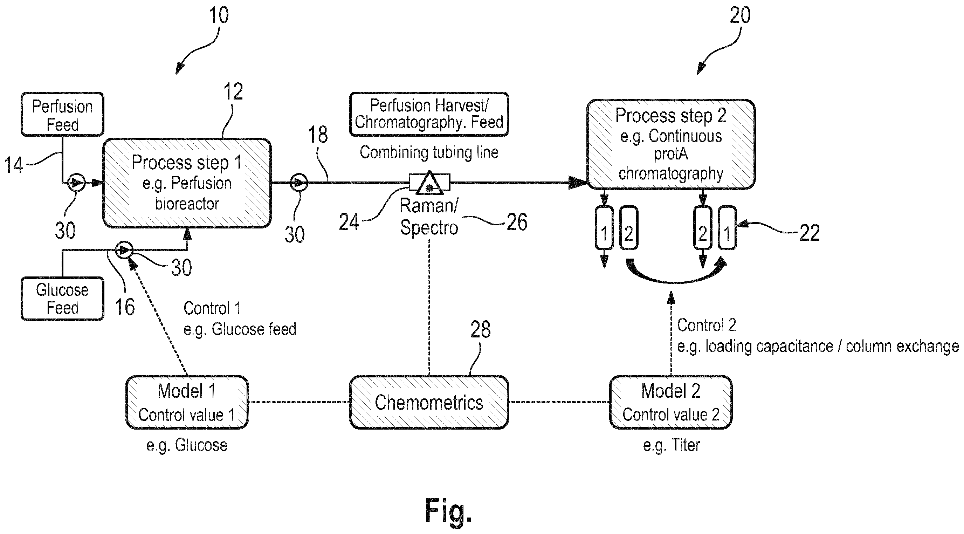

Further features and advantages of the invention will become apparent from the following description and from the accompanying drawing to which reference is made. In the drawing the only Figure shows an embodiment of the device assembly according to the invention.

In the Figure a basic setup of an integrated continuous biopharmaceutical manufacturing process is illustrated. In the present example the manufacturing process is a perfusion process including an upstream process step (first process step) and a subsequent downstream process step (second process step).

A first process equipment 10 including a bioreactor 12, such as a stirred tank or rocking motion reactor, is used for performing the upstream process step. The bioreactor 12 is connected to a pump driven perfusion feed line 14 which constantly supplies fresh medium. The bioreactor 12 is further connected to a pump driven glucose feed line 16. A harvest line, hereinafter also referred to as connecting line, 18 connects an outlet of the bioreactor 12 to a second process equipment 20 which is used for performing the downstream process step. The second process equipment 20 includes several chromatography columns 22.

A single measuring unit 24 is arranged between the first process equipment 10 and the second process equipment 20. More particular, the measuring unit 24 is a measuring cell including a flow-through measurement chamber. The measurement chamber is integrated into the harvest line 18 which simultaneously serves as a chromatography feed line. The measuring cell is part of a Raman spectrometer 26.

The Raman spectrometer 26 is connected to an evaluation and control unit 28 where algorithms based on at least two different chemometric models are stored. The evaluation and control unit 28, in turn, can automatically provide corrective feedback to both the first process equipment 10 and the second process equipment 20.

The measuring unit 24 repeatedly records a Raman spectrum (set of signals) of the liquid process medium (cell-culture medium) flowing through the harvest line 18. The spectral data are transmitted to the evaluation and control unit 28 and evaluated using a first chemometric model to determine a current value of a first parameter. In the present example, the first parameter is the current glucose concentration in the process medium that is removed from the bioreactor 12 and fed to the downstream process step. Glucose is an important nutrient that needs to be present in the broth in sufficient quantity for cell growth. However, feeding too much glucose can result in metabolic disorder and the product quality may decrease.

Based on the value of the first parameter a first corrective feedback is determined in the evaluation and control unit 28. The first corrective feedback is provided to the first process equipment 10 to achieve or maintain a target value of the first parameter. In the present example, the determined glucose concentration is used to control the pump 30 in the glucose feed line 16 to ensure a constant predetermined glucose concentration (within a given range) in the bioreactor 12.

The recorded Raman spectrum is also evaluated in the evaluation and control unit 28 using a second chemometric model to determine a current value of a second parameter. In the present example, the second parameter is the current product (target protein) concentration in the process medium removed from the bioreactor and fed to the downstream process step. Irrespective of the fact that it is important for an assessment of the upstream process step (sufficient productivity or cell growth), the value of the product concentration is also used as a control parameter in the subsequent downstream process step.

Based on the value of the second parameter a second corrective feedback is determined in the evaluation and control unit 28. The second corrective feedback is provided to the second process equipment 20. Here, the second corrective feedback is used to control the loading of the chromatography columns 22. In combination with the (known or controlled or calculated) flow rate and the (known or otherwise determined) binding capacity of the columns 22, it is possible to fully exploit the loading capacity of the columns 22 (> 80% or even > 90%) without the risk of breakthrough caused by column overload. This is a significant advantage in view of the high costs of chromatography columns 22.

It is to be noted that the steps of (i) measuring set(s) of signals of the liquid process medium at a single location, (ii) evaluating the set(s) of measured signals, (iii) determining corrective feedbacks, and (iv) controlling the first and second process steps by providing the corrective feedbacks are performed in real-time (within a specified time interval) to ensure that the continuous manufacturing process does not need to be interrupted.

The invention can be applied in many other integrated continuous pharmaceutical or biopharmaceutical manufacturing processes. Some examples are outlined below.
1. The integrated continuous biopharmaceutical manufacturing process is a mammalian cell culture perfusion process with continuous product capture, wherein
   - the first process step includes perfusion cultivation of mammalian cells in a bioreactor,
   - the first parameter is a metabolite concentration in the liquid process medium, such as a glucose concentration or a concentration of amino acids,
   - the first corrective feedback is a metabolite feed rate based on the metabolite concentration,
   - the second process step is a downstream process step including product capture with continuous Protein A column chromatography or membrane adsorption,
   - the second parameter is a product concentration in the liquid process medium, and
   the second corrective feedback is a column or a membrane adsorber loading, or a signal to switch off a column or a membrane adsorber.
   The single measurement unit is preferably arranged in the connecting line between the two process steps.
2. The integrated continuous biopharmaceutical manufacturing process is a (fed-)batch virus production process, wherein
   - the first process step includes cultivation of cells in a bioreactor,
   - the first parameter is a cell count,
   - the first corrective feedback is a feed strategy based on the cell count, preferably a metabolite bolus feed or a metabolite feed rate, or a start of infection or a multiplicity of infection,
   - the second process step is a downstream process step including virus capture and/or purification by column chromatography,
   - the second parameter is a virus count, preferably measured directly or calculated by multiplying a cell count with a ratio of virus containing cells, and
      the second corrective feedback is a column loading, or a signal to switch off a column.
   The single measurement unit is preferably arranged in the first process equipment.
3. The integrated continuous biopharmaceutical process is a virus production perfusion process with continuous virus purification, wherein
   - the first process step includes cell growth in a first bioreactor,
   - the first parameter is a metabolite concentration in the liquid process medium, such as a glucose concentration or a concentration of amino acids,
   - the first corrective feedback is a feed strategy based on the metabolite concentration,
   - the second process step includes transient infection using a second bioreactor which is constantly fed with viable cells from the first bioreactor,
   - the second parameter is a cell count,
      the second corrective feedback is a rate of volume transfer from the first bioreactor to the second bioreactor to ensure constant transfer of viable cells,
   - a third process step subsequent to the second process step includes continuous virus purification by column chromatography,
   - a third parameter is a virus and/or particle count,
   - a third corrective feedback is a column loading, or a signal to switch off a column.
   The single measurement unit is preferably arranged in the connecting line between the first and second process steps or in the connecting line between the second and third process steps.
4. The integrated continuous biopharmaceutical manufacturing process is a regenerative medicine application, wherein
   - the first process step includes cultivation of cells in a bioreactor,
   - the first parameter is a cell count,
   - the first corrective feedback is a feed strategy based on the cell count, preferably a metabolite bolus feed or a metabolite feed rate
   - the second process step is a downstream process step including cell harvest by centrifugation,
   - the second parameter is a cell differentiation obtained indirectly by measuring metabolites or directly via markers on cells, and
   - the second corrective feedback is an ideal starting time of the downstream process, or an optimization of centrifugation parameters, preferably the rotational speed of a centrifuge.

The single measurement unit is preferably arranged in the first process equipment.

### List of Reference Signs

- 10: first process equipment
- 12: bioreactor
- 14: perfusion feed line
- 16: glucose feed line
- 18: connecting line (harvest line, chromatography feed line)
- 20: second process equipment
- 22: chromatography columns
- 24: single measuring unit
- 26: spectrometer
- 28: evaluation and control unit
- 30: pump

## Claims

1. A device assembly for controlling an integrated continuous pharmaceutical or biopharmaceutical manufacturing process, the device assembly comprising:
- a first process equipment (10) adapted for performing a first process step;
- a second process equipment (20) adapted for performing a second process step subsequent to the first process step;
- a single measuring unit (24) adapted for measurement of at least one set of signals of a liquid process medium at a single location, the set of measured signals depending on at least a first parameter and a different second parameter; and
- an evaluation and control unit (28) adapted for evaluating the set of measured signals to determine a value of the first parameter and a value of the second parameter;
the evaluation and control unit (28) being further adapted for determining a first corrective feedback based on the value of the first parameter and a different second corrective feedback based on the value of the second parameter; and
the evaluation and control unit (28) being further adapted for controlling the first process step by providing the first corrective feedback to the first process equipment (10) and for controlling the subsequent second process step by providing the second corrective feedback to the second process equipment (20).

2. A device assembly for controlling an integrated continuous pharmaceutical or biopharmaceutical manufacturing process, the device assembly comprising:
- a first process equipment (10) adapted for performing a first process step;
- a second process equipment (20) adapted for performing a second process step subsequent to the first process step;
- a single measuring unit (24) adapted for measurement of at least two different sets of signals of a liquid process medium at a single location, a first set of measured signals depending on at least a first parameter and a different second set of measured signals depending on at least a different second parameter; and
- an evaluation and control unit (28) adapted for evaluating the first set of measured signals to determine a value of the first parameter and for evaluating the second set of measured signals to determine a value of the second parameter;
the evaluation and control unit (28) being further adapted for determining a first corrective feedback based on the value of the first parameter and a different second corrective feedback based on the value of the second parameter; and
the evaluation and control unit (28) being further adapted for controlling the first process step by providing the first corrective feedback to the first process equipment (10) and for controlling the subsequent second process step by providing the second corrective feedback to the second process equipment (20).

3. The device assembly according to claim 2, **characterized in that** the measuring unit (24) is adapted for simultaneous measurement of the first and second sets of signals.

4. The device assembly according to any of the preceding claims, **characterized in that** the measuring unit (24) is adapted for repeated measurement of the set(s) of signals.

5. The device assembly according to any of the preceding claims, **characterized in that** the measuring unit (24) is arranged in a connecting line (18), or in a bypass line, between the first process equipment (10) and the second process equipment (20).

6. The device assembly according to any of the preceding claims, **characterized in that** the measuring unit (24) is arranged in a harvest line (18) downstream of a bioreactor (12).

7. The device assembly according to any of the preceding claims, **characterized in that** the measuring unit (24) is arranged in the first process equipment (10), preferably in a bioreactor (12) of the first process equipment (10).

8. The device assembly according to any of the preceding claims, **characterized in that** the measurement unit is a measuring cell, preferably including a flow-through measurement chamber, which is part of a spectrometer (26), preferably based on at least one of the following spectroscopic techniques: scattering, Raman, SERS, MIR, NIR, UV/Vis, fluorescence.

9. The device assembly according to any of the preceding claims, **characterized in that** the evaluation and control unit (28) is adapted for applying a first chemometric model and a different second chemometric model to the set(s) of measured signals.

10. A method for controlling an integrated continuous pharmaceutical or biopharmaceutical manufacturing process, the process comprising at least a first process step performed in or by a first process equipment (10) and a subsequent second process step performed in or by a second process equipment (20), the method comprising the following steps:
- measuring at least one set of signals of a liquid process medium at a single location, the set of measured signals depending on at least a first parameter and a different second parameter;
- evaluating the set of measured signals to determine a value of the first parameter and a value of the second parameter;
- determining a first corrective feedback based on the value of the first parameter and a different second corrective feedback based on the value of the second parameter;
- controlling the first process step by providing the first corrective feedback to the first process equipment (10); and
- controlling the subsequent second process step by providing the second corrective feedback to the second process equipment (20).

11. A method for controlling an integrated continuous pharmaceutical or biopharmaceutical manufacturing process, the process comprising at least a first process step performed in or by a first process equipment (10) and a subsequent second process step performed in or by a second process equipment (20), the method comprising the following steps:
- measuring at least two different sets of signals of a liquid process medium at a single location, a first set of measured signals depending on at least a first parameter and a different second set of measured signals depending on at least a different second parameter;
- evaluating the first set of measured signals to determine a value of the first parameter and for evaluating the second set of measured signals to determine a value of the second parameter;
- determining a first corrective feedback based on the value of the first parameter and a different second corrective feedback based on the value of the second parameter;
- controlling the first process step by providing the first corrective feedback to the first process equipment (10); and
- controlling the subsequent second process step by providing the second corrective feedback to the second process equipment (20).

12. The method according to claim 11, **characterized in that** the first and second sets of signals are measured simultaneously during the running process.

13. The method according to claim 11 or 12, **characterized in that** the set(s) of signals are measured repeatedly during the running process.

14. The method according to any of claims 11 to 13, **characterized in that** the set(s) of signals are measured in a connecting line (18), or in a bypass line, between the first process step and the second process step.

15. The method according to any of claims 11 to 14, **characterized in that** the step of measuring set(s) of signals includes measuring a spectrum of the liquid process medium, preferably at least one of the following spectra: scattering, Raman, SERS, MIR, NIR, UV/Vis, fluorescence.

16. The method according to any of claims 11 to 15, **characterized in that** the step of evaluating the set(s) of signals includes applying a first chemometric model and a different second chemometric model to the set(s) of measured signals.
